# EUROPÄISCHE PATENTANMELDUNG

(11) **EP 3 010 253 A1**
(43) Veröffentlichungstag der Anmeldung: **20.04.2016**
(21) Anmeldenummer: 15400045.9
(22) Anmeldetag: 13.10.2015
(51) Int. Cl.: H04R 25/00, A61B 5/12

(54) **EINRICHTUNG ZUR BESTIMMUNG VON TINNITUSTÖNEN UND ZUR GENERIERUNG DERARTIGER TÖNE**

(30) Priorität: 15.10.2014 DE 102014015492
(71) Anmelder: Prignitz Mikrosystemtechnik GmbH, 19322 Wittenberge (DE); Technische Universität Dresden, 01069 Dresden (DE)
(72) Erfinder: Tymnik, Gerd, 01561 Großenhain (DE); Schönfelder, Gert, 01127 Dresden (DE); Spindler, Martin, 01099 Dresden (DE); Schlemmer, Tobias, 01159 Dresden (DE); Lippmann, Matthias, 01099 Dresden (DE); Weber, Gerhard, 01217 Dresden (DE); Schmidt, Stefan, 21514 Witzeeze (DE); Finger, Adolf, 01737 Kurort-Hartha (DE)
(74) Vertreter: Krause, Wolfgang

(57) **Zusammenfassung**

Die Erfindung betrifft Einrichtungen zur Bestimmung von Tinnitustönen und zur Generierung derartiger Töne mit mindestens einem Generator in Verbindung mit einer Steuereinrichtung und wenigstens einem Schallwandler in Kopplung an ein Ohr. Der Generator ist ein in der Frequenz durchstimmbarer und in der Phase, in der Amplitude und/oder in der Signalstruktur veränderbarer Generator.

Die Einrichtungen zeichnen sich insbesondere durch ihre individuelle Bestimmung von Tinnitustönen sowie deren Generierung und Abgabe aus.

Dazu sind im Speicher Daten von Messsignalen eines Audiogramms der Person und von dem Tinnitusgeräusch am nächsten kommenden Signalen und/oder von dem Tinnitusgeräusch entsprechenden Signalen und/oder von Pseudo-Rauschsignalen gespeichert.

Weiterhin werden die generierten Töne einzeln in der Amplitude nach der Hörkurve korrigiert und/oder durch Modulation in ihrer spektralen Breite verändert und/oder Harmonische mit dem Tinnitusgeräusch erzeugt und/oder Gemische von Tönen im Frequenzbereich als Komplex verschoben.

Bestimmte gespeicherte Daten werden als aus den Daten gewandelte Töne über den Schallwandler personenbezogen abgegeben.

## Beschreibung

Die Erfindung betrifft Einrichtungen zur Bestimmung von Tinnitustönen und zur Generierung derartiger Töne mit mindestens einem Generator im Frequenzbereich von 1 Hz bis 20 kHz in Verbindung mit einer wenigstens einen Speicher aufweisenden Steuereinrichtung und wenigstens einem Schallwandler in Kopplung an ein Ohr, wobei der Generator über die Steuereinrichtung ein in der Frequenz durchstimmbarer und in der Phase, in der Amplitude und/oder in der Signalstruktur veränderbarer Generator ist.

Durch die Druckschrift DE 30 27 791 C2 ist ein Gerät zur Behandlung des Tinnitus in Verbindung mit einem Hörgerät zum Anbringen in oder bei dem Ohr bekannt. Das Hörgerät weist ein Gehäuse mit einem Mikrophon, einem Verstärker und einem Schallgeber auf. Der Schallgeber leitet den Schall durch ein zur Einführung in das Ohr angepasstes Einführungsteil, welches den Schall mit einem Eingangsschalldruckpegel abgibt. In einem weiteren Gehäuseteil ist ein Signalgeber eines Tinnitus maskierenden Signals vorhanden, das einem Eingangsschalldruckpegel zwischen 20 und 70 dB SPL entspricht. Das Signal und dessen Ermittlung ist nicht Gegenstand dieser Druckschrift.

Die Druckschrift DE 40 14 872 A1 beinhaltet ein Tinnitus-Maskiergerät mit einem oder mehreren Signalgeneratoren, einem regelbaren Verstärker und einem oder zwei elektroakustischen Wandlern zum Umwandeln elektrischer in akustische Signale. Mindestens einer der Signalgeneratoren erzeugt ein sich kontinuierlich wiederholendes, den Hörfrequenzbereich langsam überstreichendes sinusförmiges reines Tonsignal, dessen Zyklusdauer zwischen 0,1 und 1000 s einstellbar ist. Das Maskiergerät ist auf dieses sinusförmige reine Tonsignal begrenzt.

Ein Bereitstellen von Audiosignalen für eine Tinnitustherapie ist in der Druckschrift DE 10 2012 220 620 A1 beschrieben. Grundsätzlich können beliebige Audiosignale erste Audiosignale sein. Mittels eines Hemmungsparameters wird ein objektiver Maßstab zur Verfügung gestellt, inwieweit ein zweites Audiosignal zur Therapie des Tinnitus mit der individuellen Tinnitusfrequenz des vorliegenden Einzelfalls geeignet ist. Das zweite Audiosignal wird aus dem ersten Audiosignal unter Verwendung eines Filters für einen Signalanteil des ersten Audiosignals gewonnen. Weiterhin wird dem zweiten Audiosignal ein Hemmungsparameter zugeordnet. Eine Auswahl bestimmter Audiosignale für eine personenbezogene Bereitstellung ist nicht vorgesehen.

Durch Druckschrift US 2012/0 283 593 A1 ist eine Behandlung von Tinnitus bekannt. Dabei wird ein Verfahren zum Bestimmen einer räumlichen Eigenschaft des Tinnitus angewandt, wobei der Person Testtöne aus einer Reihe von virtuellen Schallquellenpositionen im dreidimensionalen Raum präsentiert werden. Dazu wird ein Schallerzeugersystem genutzt. Die Reaktion der Person auf die Testtöne wird erfasst. Der Testton kann in seinen Eigenschaften verändert werden und wird der Person aus verschieden im Raum positionierten Schallquellen präsentiert. Das erfolgt aus einer Auswahl eines Maskierungs-Sounds und der Anpassung der Parameter. Eine Überlagerung mehrerer Signale ist dabei nicht vorgesehen.

Der im Patentanspruch 1 angegebenen Erfindung liegt die Aufgabe zugrunde, eine Einrichtung zur individuellen Bestimmung von Tinnitustönen sowie zu deren Generierung und Abgabe zu schaffen.

Diese Aufgabe wird mit den im Patentanspruch 1 aufgeführten Merkmalen gelöst.

Die Einrichtungen zur Bestimmung von Tinnitustönen und zur Generierung derartiger Töne mit mindestens einem Generator im Frequenzbereich von 1 Hz bis 20 kHz in Verbindung mit einer wenigstens einen Speicher aufweisenden Steuereinrichtung und wenigstens einem Schallwandler in Kopplung an ein Ohr, wobei der Generator über die Steuereinrichtung ein in der Frequenz frei durchstimmbarer und in der Phase, in der Amplitude und/oder in der Signalstruktur veränderbarer Generator ist, zeichnen sich insbesondere durch ihre individuelle Bestimmung von Tinnitustönen sowie deren Generierung und Abgabe aus.

Dazu sind im Speicher Daten von
- Messsignalen eines Audiogramms der Person gespeichert, wobei die Messsignale die Stützstellen des Audiogramms sind,
- dem Tinnitusgeräusch am nächsten kommende Signale gespeichert, wobei das aus einer ungeordneten Reihenfolge von Tönen bestimmte Tongemische sind,
- dem Tinnitusgeräusch entsprechende Signale gespeichert, wobei das ein dem Tinnitusgeräusch entsprechender Vergleichston, entsprechendes Tongemisch oder entsprechende Tonfolge des Generators ist, und
- Pseudo-Rauschsignale gespeichert, wobei Pseudo-Rauschsignale entweder Pseudozufallssignale oder Pseudo-Noise-Signale sind.

Die Steuereinrichtung ist eine die Daten und damit die daraus einzeln generierten Töne
- in der Amplitude nach der Hörkurve korrigierende und/oder
- durch Modulation in ihrer spektralen Breite verändernde und/oder
- Harmonische mit dem Tinnitusgeräusch erzeugende und/oder
- Gemische von Tönen im Frequenzbereich als Komplex verschiebende und
   zu einem Gesamtsignal mischende und dabei jedem mittels eines Generators generierten Signals
- die jeweilige Hörschwelle zuordnende und
- die Mindestlautstärke als Hörschwelle als Funktion der vom jeweiligen Generator generierten Frequenz und als Töne gespeicherte Frequenzen ermittelnde und
- die jeweils dazugehörigen Daten speichernde
Steuereinrichtung.

Die Steuereinrichtung ist weiterhin eine daraus
- einen Vergleichston für das betroffene Ohr,
- einen Vergleichston für das andere und nicht betroffene Ohr oder
- einen Vergleichston für das betroffene Ohr und ein Rauschsignal für das andere Ohr bereitstellende Steuereinrichtung.

Die Steuereinrichtung, der Generator und der Schallwandler sind so miteinander verbunden, dass bestimmte gespeicherte Daten als aus den Daten gewandelte Töne und/oder Tongemische über den Schallwandler personenbezogen abgegeben werden.

Hören-Denken-Sprechen bilden ein komplexes System des Menschen, die einander bedingen. Die menschliche Sprache ist ein Kommunikationsmittel von außerordentlicher Komplexität, Anpassungsfähigkeit und direkter Wirksamkeit. Spracherzeugung und Sprachwahrnehmung sind zusammengehörende Teile, die im Verlauf der Evolution optimal aufeinander abgestimmt worden sind. Das wesentliche Kriterium war dabei, hohe störungsarme Verständlichkeit bei gleichzeitig minimalem Aufwand zu erreichen. Die Frequenzgänge des Ohres und der Sprache sind dabei ideal aufeinander abgestimmt. Die Kommunikationskette für den lautsprachlichen zwischenmenschlichen Informationsaustausch ist jedoch auf allen Ebenen störanfällig. Evolutionsbiologisch kann die Sprache als Spezialisierung der Musik verstanden werden.

Eine wesentliche und entscheidende Aufgabe des Hörsinnes war jedoch, als akustisches Warnorgan zu fungieren. Fremde Geräusche mussten in Echtzeit rechtzeitig erkannt und zugeordnet werden. Über den Hörsinn, der selbst im Schlaf nicht komplett abzustellen ist, wird der Körper in Alarmbereitschaft versetzt.

Hier liegt auch der Knackpunkt beim Tinnitus. Ein relativ harmloser körperintern produzierter und perzeptierter Ton kann den Betroffenen in panischen Zustand versetzen. Das hängt natürlich von der psychischen Konstitution des davon betroffenen Menschen ab. Dieser akustischen Störung kann der Patient nicht entrinnen.

Als Tinnitus wird eine Hörwahrnehmung definiert, die nicht durch äußere akustische Reizung verursacht wird oder dadurch entsteht. Es ist davon auszugehen, dass ungefähr bei der Hälfte der Betroffenen ein tonaler Tinnitus vorliegt. Die andere Hälfte leidet an einem Rauschen als ein Gemisch von Tönen. Tinnitus ist somit das häufigste Symptom in der HNO-Heilkunde (HNO für Hals-Nase-Ohr). Jedoch verfügt der HNO-Arzt über keine scharfe therapeutische Waffe gegen den Tinnitus. Er ist faktisch machtlos. Dennoch gehören Diagnostik, Differentialdiagnostik und Therapie in die Hand des erfahrenen HNO-Arztes.

Der Tinnitus ist derzeit objektiv nicht messbar. Es kann als Arbeitshypothese eine Klassifikation in den Mittelohrtinnitus, den cochleären Tinnitus und den zentralen Tinnitus erfolgen.

Der Mittelohrtinnitus wird durch unterschiedliche Erkrankungen des Gehörganges und des Mittelohres verursacht. Zu beheben ist er durch konservative oder operative HNO-Maßnahmen. Der cochleäre Tinnitus präsentiert sich dem HNO-Arzt am häufigsten als akuter Tinnitus ohne erkennbare Ursache. Mit hoher Wahrscheinlichkeit handelt es sich um eine spontane Störung der Innenohrfunktion, meist ausgelöst durch eine passagere Durchblutungsstörung des Innenohres als Stressfolge oder infolge Lärmtrauma.

Das fein abgestimmte Hören wird durch ein Wechselspiel zwischen inneren (eine Reihe) und äußeren (drei Reihen) Haarzellen ermöglicht. Dabei sind die inneren Haarzellen die eigentlichen Hörzellen und die äußeren Haarzellen die Verstärker- und Pufferzellen. Die Stereocilien (feine Härchen) der äußeren Haarzellen sind mit einer Deckplatte (Membrana tectoria) verwachsen. Sie sind somit ruhig gestellt. Werden diese Stereocilien von der Membrana tectoria abgekoppelt, beispielsweise durch Lärmeinwirkung oder nicht ausreichender Sauerstoffversorgung, dann gibt es eine Dauererregung der inneren Haarzellen. Ein Dauersignal (Tinnitus) entsteht ohne tatsächliche akustische Reizung.

Der zentrale Tinnitus wird in eine primäre und sekundäre Form unterschieden. Bei der primären Form ist der Hörnerv direkt geschädigt. Hierbei entsteht ein Dauersignal. Ein entsprechender Tinnitus wird wahrgenommen. Der sekundäre zentrale Tinnitus ist analog dem Phantomschmerz zu sehen. Ein ursprünglich peripherer Tinnitus kann sich zentralverankern und somit verselbständigen. Bei dem chronischen Tinnitus wirken keine Medikamente mehr. Die Behandlung wird zunehmend von Psychologen und Psychotherapeuten übernommen und dominiert.

Es ist davon auszugehen, dass der perzipierte (virtuelle) Tinnitus-Schall fehlerhaft im Innenohr generiert (cochleärer Tinnitus) oder fehlerhaft im akustischen Informationssystem abgebildet wird. Dabei reicht die Vielfalt der Wahrnehmung des Tinnitus von reinen Sinustönen bis hin zu statischen und zeitlich dynamischen breitbandigen Rauschsignalen. Der in der Cochlea generierte Tinnitus ist durch die Parameter Frequenz und Phase oder Frequenzgang und Phasengang gekennzeichnet. Frequenz und Intensität des Tinnitus-Schalles können subjektiv bestimmt werden. Der zentrale Tinnitus stellt sich dem Patienten als Äquivalent eines realen Schalles dar. Auch hier können Parameter wie Frequenz und Lautstärke bestimmt werden, wobei die subjektive Lautstärke als von der Beschaffenheit der real gehörten Geräusche abhängig zu betrachten ist.

Eine besondere Herausforderung für die Messung der Tinnitusfrequenz stellt die Vielzahl der Übertragungskanäle von der Cochlea zum Gehirn da. Das menschliche Ohr besitzt mehr als 3000 innere und entsprechend viele äußere Haarzellen, die jeweils für eine eigene Frequenz optimiert sind. Theoretisch kann eine einzelne defekte Haarzelle einen Tinnitus auslösen.

Die Einrichtung zeichnet sich dadurch aus, dass eine Tinnitusunterdrückung bei Menschen mit einem chronischen und nicht behandelbaren Tinnitus erfolgt. Das erfolgt über eine individuelle Einstellung der Töne, die zur Tinnitusunterdrückung über die Schallwandler abgegeben werden. Dies führt zu einer temporären, über den Zeitraum der Beschallung hinausgehenden Aufhebung des Tinnitusgeräusches, wobei der Zeitraum der Wirkung von Patient zu Patient verschieden und nicht vorhersagbar ist. Grundlage bildet dabei das individuell ermittelte Audiogramm des Menschen. Damit ist der Ton des individuellen Tinnitsgeräusches einfacher und vor allem schneller zu ermitteln. Das kann innerhalb beispielsweise von 40 Minuten erfolgen, so dass ein Ermüden und damit einhergehende Fehleinschätzungen vermieden werden können. Die Aufnahme des Audiogramms kann auch jederzeit gestoppt und danach fortgesetzt werden. Ein weiterer Vorteil besteht darin, dass sowohl einzelne Töne also Signale mit einzelnen Frequenzen als auch Tongemische also Signale mit einem Frequenzgemisch einfach entsprechend des Tinnitusgeräusches generierbar sind. Diese Prozedur wird für jedes Ohr einzeln und/oder für beide Ohren gemeinsam durchgeführt. Bei Letzterem wird ein Ohr definiert mit einem Ton beaufschlagt, so dass Fehleinschätzungen bei der Bestimmung des Tinnitusgeräusches ausgeschlossen werden können.

Vorteilhafte Ausgestaltungen der Erfindung sind in den Patentansprüchen 2 bis 9 angegeben.

Die Steuereinrichtung ist nach der Weiterbildung des Patentanspruchs 2 eine die dem Tinnitusgeräusch am nächsten kommenden Signale und/oder die dem Tinnitusgeräusch entsprechenden Signale und/oder Pseudo-Rauschsignale der Hörkurve zuordnende und daraus die Lautstärke korrigierende Steuereinrichtung. Das erfolgt mittels der Steuereinrichtung auch bei Tonkombinationen, harmonischen Tonkombinationen und/oder Oberwellen.

Die Steuereinrichtung ist nach der Weiterbildung des Patentanspruchs 3 eine als Tinnitusgeräusch ermittelten Messsignalen mittels spektraler harmonischer Tonkombinationen jeweils einen harmonischen Klang oder mehrere harmonische Klänge mit und/oder ohne hörbare Grundfrequenz mit dem Tinnitusgeräusch erzeugende und als Daten speichernde Steuereinrichtung. Dadurch kann eine bessere Selektion des Tinnitusgeräusches erfolgen.

Die Steuereinrichtung ist nach der Weiterbildung des Patentanspruchs 4 eine als Tinnitusgeräusch ermittelten Messsignalen daraus jeweils eine harmonisierte Tonleiter berechnende und als Daten speichernde Steuereinrichtung. Harmonische Töne werden angenehmer wahrgenommen. Das Harmonieempfinden ist besser ausgeprägt als das absolute Tonempfinden.

Die Steuereinrichtung ist nach der Weiterbildung des Patentanspruchs 5 eine ein Musikstück oder eine Tonfolge auf das Tinnitusgeräusch transformierende und/oder harmonisierende und jeweils als Daten speichernde Steuereinrichtung. Dabei können Musikstücke, Tonfolgen und Tonkombinationen zur Therapie des Tinnitus verwendet werden.

Das Musikstück oder die Tonfolge sind nach der Weiterbildung des Patentanspruchs 6 in der Steuereinrichtung gespeicherte Audiodaten und/oder eine formelle Beschreibung.

Das Pseudo-Rauschsignal ist nach der Weiterbildung des Patentanspruchs 7 eine Ableitung einer getakteten Pseudo-Zufalls-Maximallängen-Codesequenz eines mit der Steuereinrichtung verbundenen Pseudo Zufallsgenerators. Vorteilhafterweise können frequenzmäßig selektierte Bereiche des Rauschens bei der Therapie des Tinnitus verwendet werden.

Die Steuereinrichtung ist nach der Weiterbildung des Patentanspruchs 8 eine das Pseudo-Noise-Signal mit wenigstens einem harmonischen Signal verknüpfende und als Daten speichernde Steuereinrichtung.

Dem Pseudo-Zufallsgenerator ist nach der Weiterbildung des Patentanspruchs 9 vorteilhafterweise eine Filtereinrichtung zur Selektion und/oder Modulation und/oder Strukturwandlung des Pseudo-Noise-Signales in Amplitude und/oder Phase und/oder Frequenzspektrum nachgeschaltet.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Bestimmung von Tinnitustönen und zur Generierung derartiger Töne in einer Blockschaltung,
- Fig. 2: eine weitere Einrichtung mit einem zusätzlichen Soundgenerator in einer Blockschaltung und
- Fig. 3: ein Soundgenerator in einer Blockschaltung.

Eine Einrichtung zur Bestimmung von Tinnitustönen und zur Generierung derartiger Töne besteht im Wesentlichen aus mehreren Generatoren 9, Endstufen 11, 12 zur Verbindung mit einem Kopfhörer 13 als Schallwandler, einer Steuereinrichtung als zentrale Datenverarbeitungseinheit 14 in Verbindung mit einem Speicher 17 mit Systemdaten, einem Speicher 18 mit Nutzer- und Audiodaten, einem Datensichtgerät 15 als ein Bildschirm, einer Tastatur 16 und einem Interface 19. Die Datenverarbeitungseinheit 14 und die Speicher 17, 18 sind Bestandteile eines bekannten Datenverarbeitungssystems oder einer bekannten Frei-Programmierbaren-Logikeinheit.

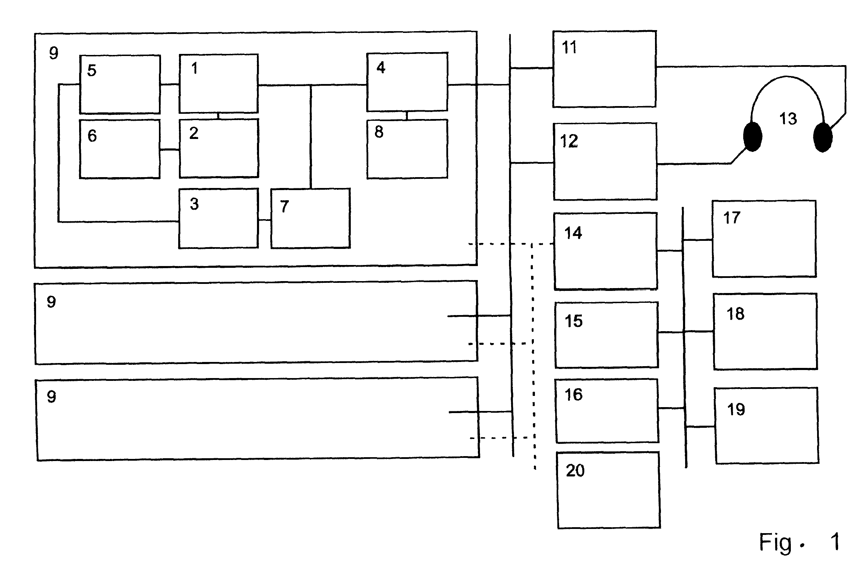

Die Fig. 1 zeigt eine Einrichtung zur Bestimmung von Tinnitustönen und zur Generierung derartiger Töne in einer prinzipiellen Blockschaltung.

Jeder der Generatoren 9 besteht aus einem frei durchstimmbaren Frequenzgenerator 1 im Frequenzbereich von 1 Hz bis 20 kHz, der über einen Lautstärkeregler 4 mit den Endstufen 11, 12 verbunden ist. Die Schritte sind dabei kleiner oder gleich 1 ct. Der Lautstärkeregler 4 ist mit einem Begrenzer 8 verbunden, so dass die audiologisch als unschädlich deklarierten Grenzen nicht überschritten werden können. Weiterhin ist ein mit einem ersten Modulator 6 verbundener Phasengenerator 2 mit dem Frequenzgenerator 1 zusammengeschaltet, so dass der Frequenzgenerator 1 ein über den ersten Modulator 6 und den Phasengenerators ein in Phasen modulierbarer Frequenzgenerator 1 ist. Darüber hinaus ist der Frequenzgenerator 1 ein über einen Filter 7 am Ausgang des Frequenzgenerators 1, einen Rauschgenerator 3 und einen zweiten Modulator 5 modulierbarer Frequenzgenerator 1. Damit ist der Generator 9 ein in der Frequenz durchstimmbarer und in der Phase, in der Amplitude und/oder in der Signalstruktur veränderbarer Generator 9.

Die Endstufen 11, 12 sind eine Endstufe 11 für das linke Ohr und eine Endstufe 12 für das rechte Ohr. Dazu sind die Endstufen 11, 12 mit dem Kopfhörer 13 als Schallwandler verbindbar.

Die Einrichtung befindet sich in einem Gehäuse. Die Inbetriebnahme und Ermittlung und Speicherung der einzelnen Daten zur Tinnitusunterdrückung erfolgt mittels einer Menüführung und Eingaben über die Tastatur 16.

Mit der realisierten Einrichtung zur Bestimmung von Tinnitustönen und zur Generierung derartiger Töne ergibt sich die folgende Arbeits- und Funktionsweise.

Die Aufnahme eines Audiogramms erfolgt mittels der Wahl bestimmter Werte wie folgt:
- Geschwindigkeit der Audiogrammaufnahme
   Für die Aufnahme der Audiogramme steigt oder fällt die Lautstärke über eine Zeit, was auch teilweise durch den Nutzer steuerbar ist. Die Geschwindigkeit der Änderung, und damit die Geschwindigkeit der Audiogrammaufnahme, ist einstellbar.
- Trennton im Audiogramm
   Zwischen den Audiogramm-Punkten kann ein Trennton ausgegeben werden. Erfolgt die Datenerfassung in der Umgebung des Trenntons, wird automatisch eine Ausweichfrequenz genutzt, welche auch einstellbar ist.
- Lautstärke des Trenntons
   Die Frequenz des Trenntons als Zwischenton ist auf 1000 Hz eingestellt. Das ist eine gut hörbare Frequenz, mit der vorteilhaft eine Lautstärke gewählt werden kann. Die Einstellung der Lautstärke erfolgt über die Tastatur. Natürlich kann hierbei auch die Frequenz über die Tastatur verändert werden.
- Verbesserung der Hörbarkeit des Audiogramms
   Um bei der Aufnahme der Audiogramme eine bessere Hörbarkeit zu erreichen, kann dem Ton eine Modulation hinterlegt werden.
- Aufnahme des Audiogramms
   Bei der Aufnahme der Audiogramme erfolgt eine automatische Änderung der Lautstärke. Diese Geschwindigkeit lässt sich auf das individuelle Tempo anpassen. Der Wert hat keine Skalierung. Die Steuerung erfolgt über die Tastatur.
- Lautstärke bei der Aufnahme des Audiogramms
   Für die Programme zur Tinnitus-Suche wird das Ton-Signal oberhalb der gemessenen Hörkurve gehalten und damit Schwankungen im Hörvermögen ausgeglichen. Wie viel lauter als die Hörschwelle der Ton ist, kann über die Tastatur numerisch eingegeben werden.

Zur Erstellung von Audiogrammen sind mehrere Varianten mit unterschiedlicher Anforderung implementiert. Die Aufnahme der Hörkurve erfolgt dazu an 8, 25, 48, 96 oder mehr Frequenzen. Die Funktionen zur Tinnitusbestimmung erfordern mindestens 48 Punkte. Das Zoom-Audiometer erlaubt die Aufnahme von Spezial-Audiogrammen in einem feinen Bereich mit variablen Frequenzen.

Alle Funktionen der Varianten zur Erstellung von Audiogrammen arbeiten unter der gleichen Bedienoberfläche mit den Schritten der Wahl einer Audiometer-Funktion und dem Vertäuben des jeweils anderen Ohrs.

Wenn der Tinnitus auf beiden Seiten vorhanden ist, lenkt dies bei der Aufnahme der Hörkurve oder der Tinnitussuche ab. Aus diesem Grund wird auf dem nicht gemessenen Ohr ein Rauschen eingespielt. Die Lautstärke des Rauschens kann mit den Lautstärke-Tasten der Tastatur verändert werden.

Die Audiogrammaufnahme beginnt mit dem rechten Ohr. Sie kann jeder Zeit abgebrochen werden. Soll die Aufnahme nur zeitweise unterbrochen werden, kann dies mit der Pausentaste erfolgen. Es kann jederzeit fortgesetzt werden. Der Fortschritt wird als Balkenanzeige des Bildschirms angezeigt.

Die Aufnahme des Frequenzpunktes beginnt mit dem Drücken einer Taste der Tastatur, wodurch die Lautstärke erhöht wird. Dies sollte bis zur deutlichen Wahrnehmung des Tones erfolgen. Je nach Einstellung kann der Ton dabei kontinuierlich oder pulsierend sein. Ist eine deutliche Wahrnehmung erreicht, wird die Taste los gelassen bis der Ton nicht mehr zu hören ist. Wenn er (gerade) verschwindet ist wieder eine Taste zu drücken bis er wieder gut wahrgenommen wird. Dieser Vorgang wiederholt sich drei Mal. Nach dem vierten loslassen der Taste ist der Vorgang beendet, was durch einen kurzen Trennton signalisiert wird. Danach wiederholt sich der Vorgang für jede Frequenz. Die Lautstärke des folgenden Tones wird automatisch unterhalb des aktuellen Tones eingestellt. Sollte dieser bereits hörbar sein, so muss mit einem kurzen Anstieg begonnen und anschließend gewartet werden, bis der Ton verschwindet.

Wenn die erfassten Werte durch Ablenkung zu weit auseinander liegen, wird die Messung für ungültig erklärt und muss wiederholt werden. Dieser Fall wird mit einem doppelten Trennton und einer Bilddarstellung des Bildschirms angezeigt.

Nachdem alle Testfrequenzen für ein Ohr abgearbeitet sind, wiederholt sich der Ablauf für das andere Ohr in gleicher Weise.

Voraussetzung für eine erfolgreiche Behandlung ist die genaue Bestimmung der Tinnitus-Frequenz. Um dies zu erreichen können mehrere Methoden angewandt werden. Alle Verfahren beruhen auf dem subjektiven Vergleich des eigenen Tinnitus-Tones mit einem angebotenen Ton. Dafür ergeben sich drei Grundvarianten:
a) der Vergleichston wird auf dem betroffenen Ohr angeboten,
b) der Vergleichston wird auf dem anderen Ohr angeboten,
c) der Vergleichston wird auf dem betroffenen Ohr angeboten und das andere Ohr mit einem Rauschsignal belegt oder
d) der Vergleichston wird auf beiden Ohren angeboten.
Die Variante c) ist vor allem erforderlich wenn beide Ohren betroffen sind.

Die Tinnitusfrequenz ist in vielen Fällen nicht mit vertretbarem Aufwand physikalisch messbar. Als universelles Messverfahren der Frequenz eines beliebigen Tinnitus bietet sich deshalb ein Suchverfahren an.

Die Einrichtung ist für die Methoden der monotonalen statistischen Suche, der monotonalen iterativen Suche und der multifrequenten Suche ausgelegt.

Die Funktionen zur Tinnitusbestimmung setzen ein Audiogramm mit mindestens 48 Punkten voraus.

In einer ersten Ausführungsform der Suche des Tinnitus kann eine statistische Suche des monotonalen Tinnitus erfolgen. Für die Bestimmung der Tinnitus-Frequenz werden 32 Töne in ungeordneter Reihenfolge mehrmals angeboten. Der Patient muss dazu entscheiden, ob der gehörte Ton höher oder tiefer als der Tinnitus ist. Das erfolgt mittels Betätigen von Tasten der Tastatur.

Nach dem kompletten Durchlauf werden die Daten ausgewertet. Dabei kann ein unsicher zugeordneter Ton wiederholt werden. Bei einer Unsicherheit kann der Ton über die Tastatur lauter oder leiser gestellt werden. Der Ton kann auch aus- oder eingeschaltet werden. Mit der nächsten Frequenz wird er automatisch wieder eingeschaltet.

Als Ergebnis der Auswertung wird der Bereich angezeigt, in welchem sich der Tinnitus wahrscheinlich befindet.

Aus dem Ergebnis der Messung wird eine neue Frequenzliste berechnet. Diese hat dann einen kleineren Bereich in dem sich der Ablauf wenigstens einmal wiederholen kann.

Die Frequenzabstände werden immer kleiner. Bei Unsicherheiten ist Mut zur Entscheidung gefragt. Da es sich um ein statistisches System handelt, entscheidet am Ende die Summe aller Eingaben. Das Ergebnis der Messung ist die Tinnitus-Frequenz, welche in das Therapieprogramm übergeben wird.

In einer zweiten Ausführungsform der Suche des Tinnitus kann eine iterative Suche des monotonalen Tinnitus erfolgen. Dazu wird auf einem Ohr ein Vergleichston angegeben, welcher so genau wie möglich auf die Frequenz des Tinnitus einzustellen ist. Die Frequenz wird dabei mit verschiedenen Tasten für jeweils andere Feinheitsgrade, beispielsweise grob/fein oder Oktave/Halbton/10 ct/1 ct, verstellt. Werden beide Töne als gleich empfunden, wird die Suche mittels Bedienung einer Taste abgeschlossen. Während der Suche kann die Lautstärke verändert und der Ton ein- und ausgeschaltet werden.

Es werden beispielsweise drei Mal acht Versuche durchgeführt, wobei der Frequenzbereich immer mehr eingeschränkt wird. Das Ergebnis der Messung ist die Tinnitus-Frequenz, welche in das Therapieprogramm übergeben wird.

Das Messverfahren der multifrequenten Suche unterscheidet sich von der monotonalen mit einer einzigen Frequenz dadurch, dass dem Patienten ein Geräusch präsentiert wird, das nach speziellen Mustern aus mehreren Tönen zusammengesetzt und mit der iterativen Suche oder Messmethode verwendet wird. Die Frequenzzusammensetzung wird dabei der Tonhöhe angepasst. Eine wichtige Klasse dieser Geräusche sind Töne ohne hörbaren Grundton. Diese werden so ausgewählt, dass der Suchraum stark eingeschränkt wird. Das Ergebnis der Suche ist eine von mehreren gleichwertigen Einstellungen. Mehrere Messungen mit unterschiedlichen Tönen werden zu einem Ergebnis zusammengefasst, das dann noch einmal mit einem monotonalen Signal verifiziert wird. Durch die starke Einschränkung des Suchraumes während der Einzelmessungen ergibt sich insgesamt eine Beschleunigung der Gesamtmessung. Je nach Kondition des Patienten und Anwendungszweck kann die gesparte Zeit zur Entlastung des ersteren oder zur Erzielung höherer Messgenauigkeiten genutzt werden.

Das Ziel der Datenerfassung ist die Therapie des Tinnitus mittels der Einrichtung. Das erfolgt mit dem Anlegen eines Kompensationstones an das betroffene Ohr. Die Daten werden dabei aus den Messprogrammen generiert.

Vor der ersten Benutzung wird die Behandlungsdauer eingestellt. Das kann durch eine numerische Eingabe über die Tastatur erfolgen. Als Dauer sind beispielsweise sechs Minuten zu empfehlen. Der Wert muss einmalig eingegeben werden und wird gespeichert.

Nach dem Start des Therapieprogramms wird der momentane Status des Tinnitus, wie schmerzhaft/störend/unangenehm, abgefragt und über die Tastatur eingestellt.

Da im Alltag nicht immer die Möglichkeit besteht, eine Therapie durchzuführen, wenn es wünschenswert wäre, kann angegeben werden, seit wie vielen Stunden es notwendig wäre. Aus diesen Zeitangaben und den Behandlungs-Zeitpunkten kann das System ermitteln, wie lange keine Beschwerden auftreten und wie sich diese Zeit verändert.

Mit dem anschließenden Start des Therapieprogramms wird der festgelegte Ton je nach Auswahl auf den rechten oder linken Kopfhörer gegeben. Die Lautstärke entspricht den ermittelten Werten, kann aber mit der Tastatur verändert werden. Während der Therapie wird die verbleibende Behandlungszeit auf dem Display angezeigt und regelmäßig aktualisiert. Nach Ablauf der gewählten Zeitspanne wird der Ton automatisch abgeschalet. Das Programm kann auch vorzeitig beendet werden.

Nach Ende des Therapieprogramms erfolgt erneut die Abfrage des Schmerzstatus als Vergleich zum Zustand vor der Behandlung.

Die Einrichtung ist weiterhin so ausgebildet, dass weitere Funktionen vorhanden sind.

Die Frequenzgeneratoren können als Tongeneratoren können frei zugänglich gemacht werden. Sie erlauben damit Funktionstests des Gerätes und eigene Anwendungen des Patienten oder Therapeuten.

Die erste Generatorfunktion ermöglicht die Ausgabe von Tönen der individuell bestimmten mikrotonalen Tinnitustonleiter. Die Tinnitustonleiter beschreibt dabei ein Tonsystem, bei dem die einzelnen Töne so verstimmt sind, dass sie eine besonders harmonische Beziehung zur Tinnitusfrequenz eingehen. Die Tonleiter erstreckt sich über den gesamten Hörbereich. Im Einzelfall muss diese Tonleiter nicht oktavperiodisch sein. Das heißt ein Intervall (die relativen Frequenzproportionen von jeweils Tönen in gleichartigem Abstand) kann je nach Oktavlage unterschiedlich groß sein.

Es wird dabei Frequenz und Bezeichnung des Tones sowie dessen Lautstärke angezeigt. Mit Tasten der Tastatur kann die Lautstärke in 1 dB- oder 10 dB-Schritten verändert werden. Mittels weiterer Tasten erfolgt die Veränderung der Tonhöhe. Dabei kann das Veränderungsintervall unterschiedlich gewählt werden. Die Auswahl, ob die Änderung auf den rechten, linken oder beiden Kanäle erfolgt, wird mit der Tastatur bestimmt und angezeigt.

Dazu kann die Grundlautstärke an die Hörkurve angepasst werden. Sinnvollerweise sollte sie auf 10 dB über der jeweils individuellen Hörschwelle der Hörkurve eingestellt sein, wodurch bei einer Frequenzänderung die empfundene Lautstärke gleich bleiben sollte. Die Lautstärkeeinstellung stellt hier eine Korrektur der Grundeinstellung dar.

Im Gegensatz zur Tongeneratorfunktion sind die den Generatoren der freie Generator nicht an die Tonleiter gebunden. Es können beliebige Frequenzen im Abstand von weniger als 1 ct eingestellt werden. Mit der Tastatur kann der markierte Wert verändert werden. Durch die Umschalttaste kann eine größere Schrittweite eingestellt werden. Eine direkte Eingabe von Werten kann ebenfalls erfolgen. Dabei kann zwischen Frequenz-Lautstärke-Phase und zwischen den Generatoren 9 gewechselt werden.

Ein Ton kann auch mit exponentiell abfallender Lautstärke bereitgestellt werden, wobei Frequenz und Abstand der Teiltöne einstellbar sind. Die Tonauswahl erfolgt im Verlauf der Tonleiter.

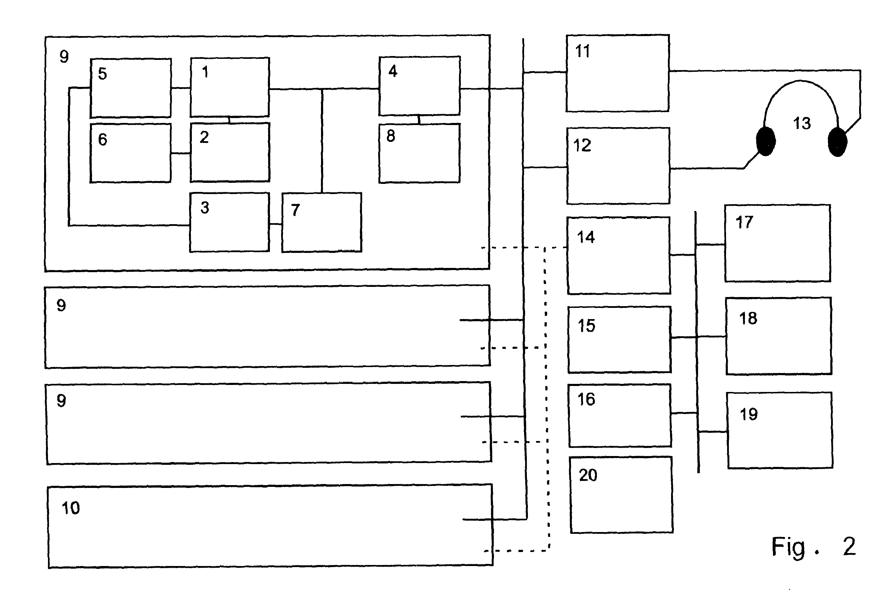

Die Fig. 2 zeigt eine Einrichtung mit einem zusätzlichen Soundgenerator 10 in einer prinzipiellen Blockschaltung.

Die Einrichtung weist in einer weiteren Ausführungsform neben den Generatoren 9 wenigstens einen weiteren Soundgenerator 10 auf. Dieser ist geeignet zusammengesetzte Tonkombinationen oder Musikstücke wieder zu geben. Diese können dabei in der Einrichtung zur Bestimmung von Tinnitustönen und zur Generierung derartiger Töne oder in einem externen und mit der Einrichtung verbindbaren Gerät an die Tinnitusfrequenzen angepasst werden.

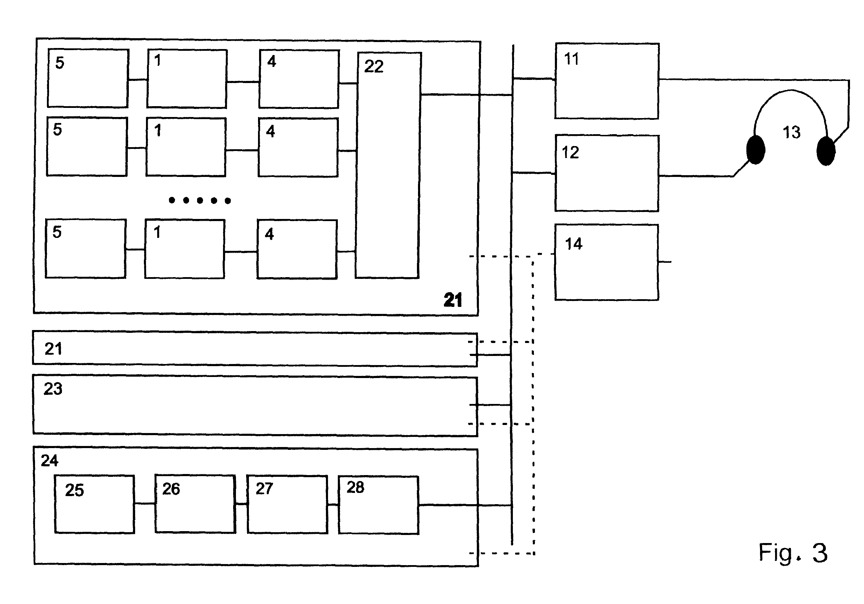

Die Fig. 3 zeigt einen Soundgenerator 10 in einer prinzipiellen Blockschaltung.

Der Soundgenerator 10 kann beispielhaft eine der folgenden Teilfunktionen implementieren:
- ein komplexer Soundmischer 21, welcher gezielt Tonkombinationen oder Töne mit einem definierten Oberwellenspektrum generieren kann,
- ein komplexes wenigstens einen Datenspeicher aufweisendes Soundgerät 23, beispielsweise ein bekannter MIDI (Musical Instrument Digital Interface)-Sequenzer oder MP3-Player,
- eine Einrichtung 24 zur Wiedergabe gespeicherter Musikstücke oder Tonsequenzen oder
- eine Erzeugung parametrisierter Pseudorauschsignale und pseudozufälliger Signale.

Pseudozufällige Signale sind definiert als deterministische Signale, bei denen bestimmte Eigenschaften hinreichend ähnlich zu den Eigenschaften echter zufälliger Signale sind. Diese Eigenschaften sind Wahrscheinlichkeiten, Wahrscheinlichkeitsverteilung und -dichte sowie statistische Mittelwerte. Im Gegensatz zu echten zufälligen Signalen sind pseudozufällige Signale jedoch periodisch und als Folge des deterministischen Charakters jederzeit exakt reproduzierbar. Diese Reproduzierbarkeit ist eine Voraussetzung für die Anwendbarkeit beim Rauschtinnitus. Die mathematischen Grundlagen für die Dimensionierung des Codegenerators für Pseudozufällige Signale bilden die, aus Galoisfeldern abgeleiteten, irreduziblen und primitiven Polynome.

Der komplexe Soundmischer 21 besteht aus mehreren jeweils in Reihe geschalteten Modulator 5, Frequenzgenerator 1 und Lautstärkeregler 4, die mit einem Mischer 22 verbunden sind. Der Ausgang des Mischers 22 ist der Ausgang des Soundmischers 21 und gleichzeitig der Ausgang des so ausgebildeten Soundgenerators 10. Die in Reihe geschalteten Modulator 5, Frequenzgenerator 1 und Lautstärkeregler 4 sind auch die Bestandteile des Generators 9, so dass mehrere mit dem Mischer 22 verbundene Generatoren 9 einen Soundgenerator 10 darstellen. Der Soundmischer 21 kann mehrfach als Modul vorhanden sein.

Die Einrichtung 24 besteht aus einem Speicher 25, einer Steuerung 26, einem Digital-Analog-Wandler 27 und einem weiteren Lautstärkeregler 28, die miteinander zusammengeschaltet sind.

Der Soundgenerator 10 dient schwerpunktmäßig der Zusammenstellung von Tonsystemen zur Bestimmung und Behandlung des multitonalen oder Rausch-Tinnitus. Die Generatoren 9 können in weiteren Ausführungsformen auch Sound-Generatoren 10 sein.

Die Einrichtung zur Bestimmung von Tinnitustönen und zur Generierung derartiger Töne kann auch wenigstens einen Rauschgenerator aufweisen, der als Standard ein (weißes) Rauschen bereitstellt. Dieses kann für jedes Ohr getrennt in der Lautstärke verändert werden.

Zur Überprüfung der Einrichtung zur Bestimmung von Tinnitustönen und zur Generierung derartiger Töne kann eine Anzeige der Audiogrammdaten in numerischer oder graphischer Form erfolgen.

Da jeder Kopfhörer eine andere Übertragungsfunktion besitzt, kann der Einsatz zwischen mehreren Typen gewählt werden. Die Auswahl des verwendeten Kopfhörers erfolgt beim Start der Einrichtung zur Bestimmung von Tinnitustönen und zur Generierung derartiger Töne oder über ein Menü.

Die eingesetzten Generatoren der Einrichtung zur Bestimmung von Tinnitustönen und zur Generierung derartiger Töne erlauben eine Umstellung der Signalform zur Tongenerierung. Durch die frei belegbare Signaltabelle der Generatoren (Wavetable) kann die Signalform umgestellt werden. Dadurch werden andere, allgemein oberwellenbehaftete Signale bereit gestellt, welche einen angenehmeren Klang haben. Der reine Sinus hat einen sehr harten Klang, was unangenehm sein kann. Bevorzugt werden Kurvenformen wie Dreieck-, Sägezahn- oder unsymmetrische Dreieckschwingungen generiert. Eine Änderung der Signalform kann ebenfalls über ein Menü erfolgen.

Die Energiequelle 20 zum Betrieb der Einrichtung ist eine Batterie oder ein Akkumulator.

Weiterhin ist wenigstens eine Datenausgabeeinheit mit der Steuereinrichtung verbunden. Das können eine Schnittstelle und/oder ein Funkmodul sein, so dass Daten an einen externen Computer übermittelbar sind. Darüber hinaus kann die Einrichtung darüber von einem externen Datenverarbeitungssystem ferngesteuert oder fernbedient werden. Darüber ist auch eine online-Darstellung der Funktionen und Abläufe möglich. Die zur Nutzung erforderlichen Daten sind vollständig, teilweise und/oder zeitweise im Datenverarbeitungssystem der Einrichtung gespeichert.

## Patentansprüche

1. Einrichtung zur Bestimmung von Tinnitustönen und zur Generierung derartiger Töne mit
- mindestens einem Generator (9) im Frequenzbereich von 1 Hz bis 20 kHz in Verbindung mit einer wenigstens einen Speicher aufweisenden Steuereinrichtung und
- wenigstens einem Schallwandler in Kopplung an ein Ohr,
wobei der Generator (9) über die Steuereinrichtung ein in der Frequenz frei durchstimmbarer und in der Phase, in der Amplitude und/oder in der Signalstruktur veränderbarer Generator (9) ist, **dadurch gekennzeichnet,**
**dass** im Speicher Daten von
- Messsignalen eines Audiogramms der Person gespeichert sind, wobei die Messsignale die Stützstellen des Audiogramms sind,
- dem Tinnitusgeräusch am nächsten kommende Signale gespeichert sind, wobei das aus einer ungeordneten Reihenfolge von Tönen bestimmte Tongemische sind,
- dem Tinnitusgeräusch entsprechende Signale gespeichert sind, wobei das ein dem Tinnitusgeräusch entsprechender Vergleichston, entsprechendes Tongemisch oder entsprechende Tonfolge des Generators (9) ist, und
- Pseudo-Rauschsignale gespeichert sind, wobei Pseudo-Rauschsignale entweder Pseudozufallssignale oder Pseudo-Noise-Signale sind, und
**dass** die Steuereinrichtung eine die Daten und damit die daraus einzeln generierten Töne
- in der Amplitude nach der Hörkurve korrigierende und/oder
- durch Modulation in ihrer spektralen Breite verändernde und/oder
- Harmonische mit dem Tinnitusgeräusch erzeugende und/oder
- Gemische von Tönen im Frequenzbereich als Komplex verschiebende und zu einem Gesamtsignal mischende und
dabei jedem mittels eines Generators (9) generierten Signals
- die jeweilige Hörschwelle zuordnende und
- die Mindestlautstärke als Hörschwelle als Funktion der vom jeweiligen Generator (9) generierten Frequenz und als Töne gespeicherte Frequenzen ermittelnde und
- die jeweils dazugehörigen Daten speichernde Steuereinrichtung ist,
**dass** die Steuereinrichtung eine daraus
- einen Vergleichston für das betroffene Ohr,
- einen Vergleichston für das andere und nicht betroffene Ohr oder
- einen Vergleichston für das betroffene Ohr und ein Rauschsignal für das andere Ohr
bereitstellende Steuereinrichtung ist und
**dass** die Steuereinrichtung, der Generator (9) und der Schallwandler so miteinander verbunden sind, dass bestimmte gespeicherte Daten als aus den Daten gewandelte Töne und/oder Tongemische über den Schallwandler personenbezogen abgegeben werden.

2. Einrichtung nach Patentanspruch 1, **dadurch gekennzeichnet, dass** die Steuereinrichtung eine die dem Tinnitusgeräusch am nächsten kommenden Signale und/oder die dem Tinnitusgeräusch entsprechenden Signale und/oder Pseudo-Rauschsignale der Hörkurve zuordnende und daraus die Lautstärke korrigierende Steuereinrichtung ist.

3. Einrichtung nach Patentanspruch 1, **dadurch gekennzeichnet, dass** die Steuereinrichtung eine als Tinnitusgeräusch ermittelten Messsignalen mittels spektraler harmonischer Tonkombinationen jeweils einen harmonischen Klang oder mehrere harmonische Klänge mit und/oder ohne hörbare Grundfrequenz verschiebende und/oder mit dem Tinnitusgeräusch harmonisch erzeugende und als Daten speichernde Steuereinrichtung ist.

4. Einrichtung nach Patentanspruch 3, **dadurch gekennzeichnet, dass** die Steuereinrichtung eine als Tinnitusgeräusch ermittelten Messsignalen daraus jeweils eine harmonisierte Tonleiter berechnende und als Daten speichernde Steuereinrichtung ist.

5. Einrichtung nach Patentanspruch 3, **dadurch gekennzeichnet, dass** die Steuereinrichtung eine ein Musikstück oder eine Tonfolge auf das Tinnitusgeräusch transformierende und/oder harmonisierende und jeweils als Daten speichernde Steuereinrichtung ist.

6. Einrichtung nach Patentanspruch 5, **dadurch gekennzeichnet, dass** das Musikstück oder die Tonfolge in der Steuereinrichtung gespeicherte Audiodaten und/oder eine formelle Beschreibung sind.

7. Einrichtung nach Patentanspruch 1, **dadurch gekennzeichnet, dass** das Pseudorauschsignal eine Ableitung einer getakteten Pseudo-Zufalls-Maximallängen-Codesequenz eines mit der Steuereinrichtung verbundenen Pseudo-Zufallsgenerators ist.

8. Einrichtung nach den Patentansprüchen 1 und 7, **dadurch gekennzeichnet, dass** die Steuereinrichtung eine ein Pseudo-Noise-Signal mit wenigstens einem harmonischen Signal verknüpfende und als Daten speichernde Steuereinrichtung ist.

9. Einrichtung nach Patentanspruch 7, **dadurch gekennzeichnet, dass** dem Pseudo-Zufallsgenerator eine Filtereinrichtung zur Selektion und/oder Modulation und/oder Strukturwandlung des Pseudo-Noise-Signales in Amplitude und/oder Phase und/oder Frequenzspektrum nachgeschaltet ist.
